# EUROPEAN PATENT SPECIFICATION

(11) **EP 1 654 267 B1**
(45) Date of publication and mention of the grant of the patent: **21.04.2010**
(21) Application number: 03818122.8
(22) Date of filing: 14.08.2003
(51) Int. Cl.: C07H 1/00

(54) **METHOD FOR TAGGING OF CARBOHYDRATES WITH ACTIVE METHYLENE COMPOUND**
VERFAHREN ZUM ANHEFTEN VON EINER AKTIVEN METHYLENVERBINDUNG AN KOHLENHYDRAT
PROCEDE DE MARQUAGE DE GLUCIDES A L'AIDE D'UN COMPOSE A BASE DE METHYLENE ACTIF

(43) Date of publication of application: 10.05.2006
(73) Proprietor: Korea Basic Science Institute, Daejeon-shi 305-333 (KR)
(72) Inventor: AHN, Yeong Hee, Cheongju-shi, Chungcheongbuk-do 360-210 (KR); YOO, Jong Shin, daejeon-shi 305-308 (KR); KIM, Soohyun, daejeon-shi 305-333 (KR)
(74) Representative: Thomas, Simon
(86) International application number: PCT/KR2003/001654
(87) International publication number: WO 2005/016948

(56) References cited:
- US-A- 4 785 794
- HELMUT ZINNER ET AL.: CHEMISCHE BERICHTE, vol. 92, 1959, pages 1614-1617, XP009070729
- KURT EGER ET AL.: LIEBIGS ANNALEN DER CHEMIE, 1989, pages 1049-1049, XP009070758
- KIN-ICHI TADANO ET AL.: JOURNAL OF ORGANIC CHEMISTRY, vol. 52, 1987, pages 1946-1956, XP002393784
- F. J. LOPEZ APARICIO: CARBOHYDRATE RESEARCH, vol. 69, 1979, pages 55-70, XP002393786
- F. J. LOPEZ APARICIO ET AL.: CARBOHYDRATE RESEARCH, vol. 135, 1985, pages 303-311, XP002393787
- ELI BREUER ET AL.: JOURNAL OF MEDICINAL CHEMISTRY, vol. 26, 1983, pages 30-34, XP002393785
- LAMARI F N ET AL: "Derivatization of carbohydrates for chromatographic, electrophoretic and mass spectrometric structure analysis" JOURNAL OF CHROMATOGRAPHY B: BIOMEDICAL SCIENCES & APPLICATIONS, ELSEVIER, AMSTERDAM, NL, vol. 793, no. 1, 5 August 2003 (2003-08-05), pages 15-36, XP004438966 ISSN: 1570-0232
- RAMSAY S.L. ET AL.: 'Mild tagging procedures for the structural analysis of glycans' CARBOHYDRATE RESEARCH vol. 333, no. 1, 22 June 2001, pages 59 - 71, XP004246299
- SUMIHIRO HASE: 'Precolumn derivatization for chromatographic and electrophoretic analyses of carbohydrates' J. CHROMATOGR. A vol. 720, no. 1, 12 January 1996, pages 173 - 182, XP004038357
- BIGGE J.C. ET AL.: 'Nonselective and efficient fluorescent labeling of glycans using 2-amino benzamide and anthranilic acid' ANAL. BIOCHEM. vol. 230, no. 2, September 1995, pages 229 - 238, XP003000876
- YEONG HEE AHN, JONG SHIN YOO: ANALYTICAL SCIENCES, vol. 17, 2001, pages 893-895,
- L. F. TIETZE, U. BEIFUSS: COMPREHENSIVE ORGAINC CHEMISTRY, vol. 2, 1991, pages 341-394,
- LUBERT STRYER: "Biochemistry" 1998, W.H. FREEMAN & COMPANY , NEW YORK * page 298 *

## Description

### FIELD OF THE INVENTION

The present invention relates to a method for tagging of carbohydrates with active methylene compound. Particularly, it relates to the method for tagging of carbohydrates with active methylene compound comprising the step of preparing carbohydrate conjugate in which carbohydrate and methylene compound are combined by mixing carbohydrate mixture and methylene compound under aqueous polar aprotic solvent containing amine base catalyst.

### BACKGROUND

It is well recognized that oligosaccharides have a variety of biologically important roles either as free carbohydrates or as constituents of glycoconjugates. In plants free oligosaccharides play important roles in carbon fluxes within the cells and as a regulator. Oligosaccharides bonded to proteins also influence protein stability, folding, and biological functions of glycoproteins. Intercellular recognition by proteins may be affected by the structure and nature of oligosaccharides. Thus the characterization of complex oligosaccharides obtained from a variety of biological media has become of increased importance, involving many kinds of analytical techniques.

Unfortunately, due to the restricted amount and complexity of samples obtained from biological media and the low proton affinity of oligosaccharides, there are many problems to be solved for the effective purification and the sensitive detection of oligosaccharides. In order to improve the sensitivity and the detectable mass range of oligosaccharides, derivatization methods such as reductive amination, designed to form a covalent bond with a variety of ligands containing nitrogen atoms, were introduced.

By the reductive amination method, the detection limit of derivatized oligosaccharides was improved greatly to fmol levels. But prior to analysis of the sample derivatized by this method, a purification procedure for the removal of excess reagents, such as sodium cyanoborohydride and the non-volatile organic ligand used, is necessary. This procedure sometimes is tedious and can be problematic especially in cases where restricted amounts of sample are available.

A number of documents disclose the reaction of carbohydrates at their reducing end with methylene active compounds (Knoevenagel reaction)under various reaction conditions.

Helmut Zinner et al: Chemische Berichte, vol. 92, 1959, pages 1614-1617, discloses the reaction of protected arabinose with various methylene active compounds. The reaction is conducted at 0°C in toluene and diethylamine is used as a catalyst. The reaction yields the expected unsaturated Knoevenagel products II - VII.

Kurt Eger et al: Liebigs Annalen Der Chemie, 1989, pages 1049, discloses the reaction of unprotected ribose with malononitrile in methanol at room temperature using ZnCl₂ as a catalyst. Under the reaction conditions the thus formed unsaturated compound undergoes a cyclisation reaction to yield furane derivative 1.

US-A-4 785 794 (Jo et al) 22 November 1988, describes the reaction of a methylene active compound with "aldehyde-function saccharides" in juices. The reaction is conducted at 40°C using alcohols as co-solvents and adding calcium salts. The unprotected saccharides undergo a Knoevenagel reaction followed by a condensation to give furanic polyalcohols (see column3, second paragraph).

Kin-Ichi Tadano et al: Journal of Organic Chemistry, vol. 52, 1987, pages 1946-1956, discloses the Knoevenagel reaction of protected D-ribose, D-xylose, D-arabinose, D-erythrose and dimethyl malonate to yield the corresponding unsaturated sugars (see for example Scheme 1, reaction 6 ->7). Pyridine is used as a catalyst and acetic anhydride is used as the solvent.

F. J. Lopez Aparicio: Carbohydrate Research, vol. 69, 1979, pages 55-70, describes the Knoevenagel reaction of compound 1 with methylene active compounds. Piperidine is used as a catalyst and toluene is used as the solvent.

F. J. Lopez Aparicio et al: Carbohydrate Research, vol. 135, 1985, pages 303-311, describes the Knoevenagel reaction of protected aldehydo sugars with acetylacetone in ethanol using piperidine as catalyst.

Eli Breuer et al: Journal of Medicinal Chemistry, vol. 26, 1983, pages 30-34, describes a Knoevenagel reaction of aldehydo sugars and methylene compounds in THF using TiCl₄ as a catalyst.

Lamari F N et al: "Derivatization of carbohydrates for chromatographic, electrophoretic and mass spectrometric structure analysis", Journal of Chromatography B: Biomedical Sciences & Applications, Elsevier, Amsterdam, NL, vol. 793, no. 1, 5 August 2003, pages 15-36, gives an overview of derivatization methods of carbohydrates for their liquid chromatographic and electrophoretic separation, as well as the mass spectrometric characterisation.

Ahn Y.H. et. al. Analytical Sciences, 2001, 17, pp 893-895 discloses a method of derivatising unprotected oligosaccharides having free hydroxyl groups on the sugar backbone with active methylene in an aqueous aprotic solvent containing a base.

Tietze L.F. et. al in Comprehensive Organic Synthesis, 1991, 2, pp 341-394 discloses the use of a dipolar aprotic solvent for Knoevenagel reactions.

Therefore, for more convenient and low-level detection of oligosaccharides without pre-treatment of sample, the development of new derivatization methods for oligosaccharides is desirable.

### SUMMARY OF THE INVENTION

The present invention provides a method for tagging of carbohydrates with an active methylene compound comprising the step of preparing a carbohydrate conjugate in which the carbohydrate is reacted with an active methylene compound in an aqueous polar aprotic solvent containing an amine base catalyst, wherein the carbohydrate is unprotected oligosaccharide having free hydroxy group at the sugar backbone, and has N-acetylglucosamine as a reducing sugar moiety, and wherein the carbohydrate conjugate is produced by the carbon-carbon bond formation between a reducing end of the carbohydrate and an active methylene carbon of the active methylene compound.

In the above method, aldol adduct is formed by the addition reaction between hemicaetal (aldehyde) group of carbohydrate moiety and carbon nucleophile (enolate) of active methylene compound, following elimination of water from the resulting aldol adduct to form α,β-unsaturated carbohydrate-tagged conjugates. The resulting α,β-unsaturated carbohydrate-tagged conjugate is converted to its ring closure form wherein sugar ring is formed by the conjugate addition of hydroxyl group in the carbohydrate chain under the basic conditions. In the method of the present invention, tagging reaction can be taken under the conditions, wherein many hydroxyl groups present in the carbohydrate chain of oligosaccharides are not protected. Active methylene compound playing as a carbon nucleophile can be malononitrile, alkyl malonate, α-cyanomalonate, β-ketoester etc. or the counterparts thereof bound to marcromolecule. The tagging reaction can be carried under aqueous basic condition using inorganic base or under polar aprotic solvent, such as DMF, NMP, DMSO etc., containing a portion of water and an amine base as a mild organic base catalyst. In the preferred embodiment of the present invention, method for the tagging of carbohydrates with active methylene compound and for the purification of the tagged oligosaccharides using such as carbohydrate affinity membrane or chromatographic techniques are disclosed.

### BRIEF DESCRIPTION OF THE DRAWINGS

Fig. 1 is a schematic representation of the oligosaccharide derivatives tagged with active methylene compound of the present invention.
Fig. 2 is a Positive-ion MALDI-TOF MS spectrum obtained from the partially tagged oligosaccharide (Man₅NAcGlu₂) with one of the active methylene moiety, malononitrile.
Fig. 3 is Positive-ion MALDI-TOF MS spectrum obtained from the N-linked oligosaccharide mixture tagged fully with one of the active methylene moiety, malononitrile.

### DETAILED DESCRIPTION OF PREFERRED EMBODIMENTS

The present invention relates to the method for tagging of carbobydrates with active methylene compound comprising the step of preparing carbohydrate conjugate in which carbohydrate and methylene compound are combined by mixing carbohydrate mixture and methylene compound under aqueous polar aprotic solvent containing amine base catalyst.

In the present invention, active methylene compound has the characteristics having two carbanion-stabilizing groups composed of carbonyl and/or nitrile group which can stabilize α-carbanion functioning as a nucleophile such as malononitrile, alkyl malonate, α-cyanoacetate, β-ketoester, β-ketoamide etc, and is preferred to have the structure of Chemical Formula 1. (F. Carey and R. Sundberg, "Advanced Organic Chemistry, Part B: Reactions and Synthesis, 3rd ed., Plenum Press, p.83-85).

<Chemical Formula 1> 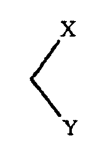 X and/or Y : CN, -CO-, -COO-, -CONH-

In the preferred embodiment of the present invention, malononitrile was used as an active methylene compound. In addition, active methylene compound can be selected from counterparts of polymers wherein each of them is bound to the solid support.

In the tagging method of the present invention, amine base catalyst can preferably be selected from a group consisting of piperidine, pyridine, pyrrolidine, sarcosine and simple amineacid. Among them, piperidine is more preferable.

In addition, polar aprotic solvent can preferably be selected from a group consisting of dimethylformamide, dimethylsulfoxide, N-methylpyrrolidinone, N-methylpiperidone and dimethoxyethane. Among them, dimethylformamide is more preferable.

In addition, carbohydrate can be selected from a group consisting of N-linked oligosaccharide and O-linked oligosaccharide of glycoprotein, and unprotected oligosaccharide having free hydroxy group at the sugar backbone is more preferable.

Carbohydrate conjugate tagged with active methylene compound prepared by the tagging method of the present invention can be purified by various purifying and analyzing methods known to the persons skilled in the art without any limitations. Said various purifying and analyzing methods can be selected from a group consisting of the method using ion exchange resin, the gel filtration method, the method using carbohydrate affinity matrix, the method using various kinds of chemically bound stationary phase and the method using adsorption medium, and the method using carbohydrate affinity matrix is more preferable for the rapid separation. In addition, aminoalkyl is preferable as a stationary phase in the chemically bound stationary phase chromatography.

By the above method, conjugate in which carbon-carbon bond is formed between reducing end of carbohydrate and active methylene carbon moiety of the active methylene compound is produced.

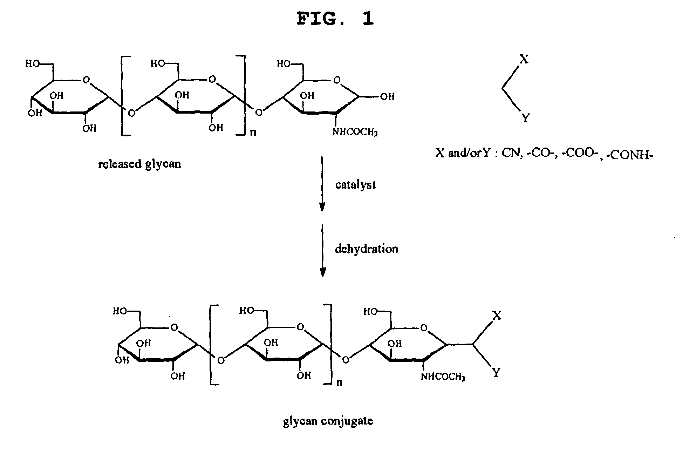

The present invention relates to a mild and efficient tagging method of oligosaccharide, mainly O-linked or N-linked, obtained from biological medium (see Fig. 1). Oligosaccharide obtained from biological medium is generally having many free hydroxyl groups on sugar backbone and restrict in the amount. Therefore, it is necessary to develop an efficient and simple tagging method without protection of many hydroxyl groups of oligosaccharide for separation and structural analysis of biologically active oligosaccharide for the use of diagnostic and industrial application.

The hemiacetal group at the reducing end of oligosaccharide is a synthetic equivalent of aldehyde. This group can react with an active methylene donor, such as malononitrile, to afford the conjugated derivative of the oligosaccharide through an addition and elimination reaction. Such an addition and elimination reaction of an simple aldehyde with an active methylene donor in anhydrous condition is known as the aldol condensation and dehydration reaction, and is a well known synthetic method in the field of organic chemistry. For efficient tagging of oligosaccharides having many free hydroxyl groups with an active methylene compound, however, aqueous conditions are inevitably necessary because of solubility of sugar. Moreover, the chemical reactivity at the reducing end of oligosaccharide can be greatly different with respect to the kind of sugar moieties present in the reducing end. For example, maltooligosaccharides having glucose unit at the reducing end of sugar chain can readily react with carbon nucleophile in aqueous basic condition. But N-acetylglucosamine terminal moiety often observed in oligosaccharides released from glycoprotein of biological media shows much less reactivity for carbon nucleophile used in the case of maltooligosaccharides, making tagging reaction to be hardly proceeded. A tagging method of free oligosaccharides, having N-acetylglucosamine as a reducing sugar moiety and unprotected many hydroxyl groups on sugar backbone, with active methylene compound as a carbon nucleophile, is described in the present invention.

In the preferred embodiment of the present invention, Man₅NAcGlu₂, as a typical oligosaccharide, is mixed with the DMF solution of malononitrile, as a representative tagging agent. To this reaction solution was added excess neat piperidine as a base and an amine catalyst. The solvent composition can be varied, but exceedingly increased portion of water can retard the progress of tagging reaction. Among several amines being exploitable as a catalyst for easy formation of Schiff base and/or as a base for inauguration of enolate formation of nucleophile, piperidine can be preferably selected. In the preferred embodiment of the present invention, the reaction tube was shaken for several seconds then incubated at temperature ranging from 4°C to 40°C. The reaction rate is affected from the kind of reducing end moiety of oligosaccharide and reaction conditions such as the composition of solvent, concentration of the samples, aliquot of the catalyst and temperature, etc.

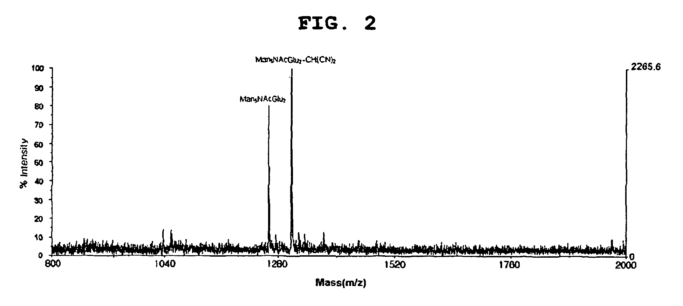

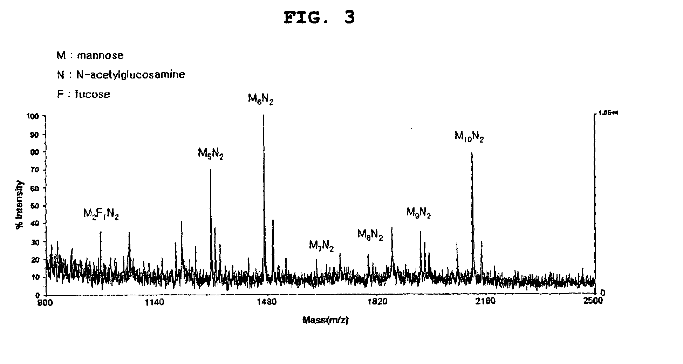

The reaction can be analyzed by matrix assisted laser desorption/ionization mass spectrometry (MALDI-MS), which is one of the powerful analytical tools for carbohydrate oligomer, having a high sensitive detectability to even impure sample. DHBA as a matrix compound can be used for the sensitive detection of the oligosaccharide-malononitrile derivative and free oligosaccharide. All signals of molecular ions obtained in this invention can be recorded as sodium ion adducts. Although several peaks due to minor impurities could also be detected, the present inventors could assign the peaks due to interesting components in the derivatization mixture (See Fig. 2 and Fig. 3).

The malononitrile tagging method can also be applied to the tagging for multi-component oligosaccharides. Oligosaccharide mixture, released from a glycoprotein is nicely tagged following the method of the present invention (See Fig. 3). The progression of the tagging reaction can be detected easily using MALDI-TOF MS. For analysis of oligosaccharides obtained from biological media, more flexible tagging condition is normally need since the purity and concentration of these sample can be variable drastically. The method of the present invention is tolerable from impurities present in sample in a wide range. The method of the present invention is also tolerable in a wide range of usage of tagging reagents from 10² equivalent to 10⁴ equivalent, and give reproducible results in the range. Additionally, the malononitrile derivative of an oligosaccharide has a highly ionizable proton at the α-position to the carbonyl or nitrile group. It has been reported that the derivatives of oligosaccharide with an active methylene compound show a high sensitivity in negative electrospray ionization (ESI) mode mass analysis.

In the second point of the present invention, the present invention provides the method for the purification of oligosaccharides tagged by active methylene compound. For tagging of a restricted amount of biological samples, excess reagent is generally used for completion of reaction or for convenience of experiment. Thus effective purification process is very important in applying the tagging method to a variety of purpose.

Although tagged oligosaccharide derivatives of the present invention have a tag moiety less polar than native oligosaccharides, they are still highly hydrophilic by virtue of oligosaccharide moiety of the tagged derivative having many hydroxy group on sugar chain. In contrast, all reagents used for the tagging reaction of oligosaccharide are non-ionic organic materials except water used as a solvent. Any kind of inorganic reagent is not required for the tagging reaction of oligosaccharide in the present invention. Thus affinity purification method exploiting the hydrophilic property due to sugar moiety of tagged samples is plausible. As an example representative, cellulose filter kit can be used for simple purification of tagged sugar. After the cellulose filter kit activated by aqueous acetic acid solution is equilibrated with acetonitrile, a portion of reaction solution is loaded on filter and equilibrated for a few minutes. Chemical reagents to be removed, such as polar aprotic organic solvents, tagging reagent and organic amine base catalysts, are washed off by a water miscible organic solvent or aqueous mixture thereof. Tagged oligosaccharide purified can be eluted with water. When active methylene compound bound to polymer or solid support is used as a tagging compound, the purification process of solid-bound tagged sugar can also be conducted simply by washing techniques with an appropriate solvent.

### EXAMPLES

Practical and presently preferred embodiments of the present invention are.illustrated as shown in the following Examples.

### Example 1: Tagging of oligosaccharide

N-linked oligosaccharide mixture obtained from glycoproteins present in the biological media by a known biological or chemical method of the prior art was dissolved in 2 µl distilled water. Malononitrile (1.0 µmol, 66 µg) was dissolved in dimethylformamide (10 µl) and was mixed with aqueous oligosaccharide solution prepared above. After piperidine (1.0 µmol, 0.1 µl) was added to the above tagging solution, the reaction tube was incubated at low temperature (10°C - 15°C) for about 3 days. A portion of tagging, solution was taken and purified.

### EXAMPLE 2 Purification of the tagged oligosaccharide

A cellulose filter was activated through washing with 5 ml of water and subsequent 30% aqueous acetic acid solution. A portion of the tagging solution of oligosaccharides prepared by the method of Example 1 was loaded onto the filter. After standing for about 10 min, the filter was washed with 5 ml of anhydrous acetonitrile. The tagged oligosaccharides was eluted from the filter with water (0.5 ml x 2) and was dried under vacuum. After dissolving lyophilized tagged oligosaccharide into distilled water, some of them were taken for MALDI MS (matrix assisted laser desorption/ionization mass spectrosmatry) analysis. DHBA (dihydroxybenzoic acid) was uses as a matrix.

As a result, all the mass spectral signals of sample compound were recorded as a sodium ion adduct (Fig. 2 and Fig. 3).

### INDUSTRIAL APPLICABILITY

As described hereinbefore, the tagging method of the present invention does not need many kinds of chemical reagent and the reactions can be taken even in the presence of certain amount of impurities. So, it can be used for the analysis of oligosaccharide present in the various kinds of samples.

## Claims

1. Method for tagging of carbohydrates with an active methylene compound comprising the step of preparing a carbohydrate conjugate in which the carbohydrate is reacted with an active methylene compound in an aqueous polar aprotic solvent containing an amine base catalyst, wherein the carbohydrate is unprotected oligosaccharide having free hydroxy group at the sugar backbone, and has N-acetylglucosamine as a reducing sugar moiety, and wherein the carbohydrate conjugate is produced by the carbon-carbon bond formation between a reducing end of the carbohydrate and an active methylene carbon of the active methylene compound.

2. The method according to claim 1, wherein the amine base catalyst is selected from the group consisting of piperidine, pyridine, pyrrolidine and sarcosine.

3. The method according to claim 2, wherein the amine base catalyst is piperidine.

4. The method according to claim 1, wherein the polar aprotic solvent is selected from the group consisting of dimethylformamide, dimethylsulfoxide, N-methylpyrrolidinone, N-methylpiperidone and dimethoxyethane.

5. The method according to claim 4, wherein the polar aprotic solvent is dimethylformamide.

6. The method according to claim 1, wherein the active methylene compound has the structure of Chemical Formula 1 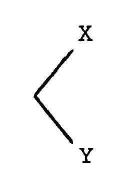 X and/or Y: CN, -CO-, -COO-, -CONH-.

7. The method according to claim 1, wherein the active methylene compound is selected from the group consisting of malononitrile, alkyl malonate, α-cyanoacetate, α-cyanoacetamide, β-ketoester, β-ketoamide and counterparts thereof wherein each of them is bound to a solid support.

8. The method according to claim 7, wherein the active methylene compound is malononitrile.

## Patentansprüche

1. Methode zum Identifizieren von Kohlenhydraten mit einer aktiven Methylenverbindung, umfassend den Schritt des Herstellens eines Kohlenhydratkonjugats, wobei das Kohlenhydrat mit einer aktiven Methylenverbindung in einem wässrigen polaren aprotischen Lösungsmittel reagiert wird, das einen Amin-Base-Katalysator enthält, wobei das Kohlenhydrat ungeschütztes Oligosaccharid ist, das eine freie Hydroxygruppe an der Zuckerrückgratkette und N-Acetylglucosamin als reduzierenden Zuckeranteil aufweist und wobei das Kohlenhydratkonjugat durch die Bildung der Kohlenstoff-Kohlenstoff-Bindung zwischen einem reduzierenden Ende des Kohlenhydrats und einem aktiven Methylenkohlenstoff der aktiven Methylenverbindung hergestellt wird.

2. Methode nach Anspruch 1, wobei der Amin-Base-Katalysator aus der Gruppe ausgewählt ist bestehend aus Piperidin, Pyridin, Pyrrolidin und Sarcosin.

3. Methode nach Anspruch 2, wobei der Amin-Base-Katalysator Piperidin ist.

4. Methode nach Anspruch 1, wobei das polare aprotische Lösungsmittel aus der Gruppe ausgewählt ist bestehend aus Dimethylformamid, Dimethylsulfoxid, N-Methylpyrrolidinon, N-Methylpiperidon und Dimethoxyethan.

5. Methode nach Anspruch 4, wobei das polare aprotische Lösungsmittel Dimethylformamid ist.

6. Methode nach Anspruch 1, wobei die aktive Methylenverbindung die Struktur der chemischen Formel 1 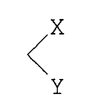 aufweist,
X und/oder Y: CN, -CO-, -COO-, -CONH- sind.

7. Methode nach Anspruch 1, wobei die aktive Methylenverbindung aus der Gruppe ausgewählt ist bestehend aus Malononitril, Alkylmalonat, α-Cyanoacetat, α-Cyanoacetamid, β-Ketoester, β-Ketoamid und Pendanten derselben, wobei jedes davon an einen festen Träger gebunden ist.

8. Methode nach Anspruch 7, wobei die aktive Methylenverbindung Malononitril ist.

## Revendications

1. Procédé de marquage de glucides avec un composé de méthylène actif, comprenant une étape de préparation d'un conjugué de glucide dans laquelle on fait réagir le glucide avec un composé de méthylène actif dans un solvant aprotique polaire aqueux contenant un catalyseur à base d'amine, le glucide étant un oligosaccharide non protégé qui présente un groupe hydroxyle libre sur l'ossature sucre et une N-acétylglucosamine en tant que fragment sucre réducteur, et dans laquelle le conjugué de glucide est produit par la formation de liaisons carbone-carbone entre une extrémité réductrice du glucide et un carbone du composé de méthylène actif.

2. Procédé selon la revendication 1, dans lequel le catalyseur à base d'amine est choisi dans le groupe constitué par la pipéridine, la pyridine, la pyrrolidine et la sarcosine.

3. Procédé selon la revendication 2, dans lequel le catalyseur à base d'amine est la pipéridine.

4. Procédé selon la revendication 1, dans lequel le solvant aprotique polaire est choisi dans le groupe constitué par le diméthylformamide, le diméthylsulfoxyde, la N-méthylpyrrolidinone, la N-méthylpipéridone et le diméthoxyéthane.

5. Procédé selon la revendication 4, dans lequel le solvant aprotique polaire est le diméthylformamide.

6. Procédé selon la revendication 1, dans lequel le composé de méthylène actif possède la structure de la formule chimique 1 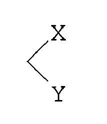 où X et/ou Y sont CN, -CO-, -COO-, -CONH-.

7. Procédé selon la revendication 1, dans lequel le composé de méthylène actif est choisi dans le groupe constitué par les composés malononitrile, alkylmalonate, α-cyanoacétate, α-cyanoacétamide, β-cétoester, β-cétamide et leurs homologues dans lesquels chacun d'eux est lié à un support solide.

8. Procédé selon la revendication 7, dans lequel le composé de méthylène actif est le malononitrile.
